# EUROPEAN PATENT APPLICATION

(11) **EP 2 168 573 A1**
(43) Date of publication of application: **31.03.2010**
(21) Application number: 08165462.6
(22) Date of filing: 30.09.2008
(51) Int. Cl.: A61K 9/20, A61K 31/397

(54) **Formulations comprising ezetimibe**

(71) Applicant: LEK Pharmaceuticals d.d., 1526 Ljubljana (SI)
(72) Inventor: The designation of the inventor has not yet been filed
(74) Representative: Opresnik, Marko

(57) **Abstract**

Formulations of ezetimibe are described, wherein attention is paid, alternatively or optionally in combination, to (i) the existence of ezetimibe in the form of primary particles, which when blended with a suitable hydrophilic excipient in particulate form are allowed adsorb on the surface of particles of the hydrophilic excipient; (ii) a selection of specific types of excipients; and/or (iii) a careful control of how the formulation of ezetimibe is obtained.

## Description

### Field of the Invention

The present invention relates to new formulations of ezetimibe, as well as a pharmaceutical formulation containing the same.

### Description of Background Art

Ezetimibe, chemically 1-(4-fluorophenyl)-3(R)-[3-(4fluorophenyl)-3(S)-hydroxypropyl]-4(S)-(4-hydroxyphenyl)-2-azetidinone, belongs to a class of lipid-lowering compounds that selectively inhibits the intestinal absorption of cholesterol and related phytosterols.

The drug is a well tolerated and effective total-C (total cholesterol), LDL-C (low density lipoprotein cholesterol), Apo B (apolipoprotein B) and TG (triglycerides) reduction agent with benefit in the secondary prevention of vascular events, such as atherosclerosis. On the other hand, ezetimibe increases HDL-C (high density lipoprotein cholesterol) or "good cholesterol" in patient with hypercholesterolemia.

US Patent 7,030,106 B2 claims a pharmaceutical composition consisting of ezetimibe, and, as excipients, lactose monohydrate (filler), microcrystalline cellulose (filler), povidone (binder), croscarmellose sodium (disintegrant), sodium lauryl sulfate (surfactant) and magnesium stearate (lubricant). EP 1 849 459 A1 describes a composition comprising ezetimibe co-milled with at least one hydrophilic excipient. Co-milling is carried out with saccharides or polysaccharides, typically starch, to obtain ezetimibe particle size of d(0.9) of less than or equal to about 25 µm, preferably less than or equal to 10 µm, and more preferably less than or equal to about 5 µm.

Further background technology is described in Kosoglou T. et al., Clin Pharmacokinet 44 (2005) 467-494; Christensen L. H. et al., Drug Dev Ind Pharm 20 (1994) 2195-2213; Inghelbrecht S. et al., Int J Pharm 171 (1998) 195-206 ; Physicians' Desk Reference, 61 st edition, Thomson PDR, NJ, USA, 2007, pp. 2120-2125; and Sharma S. et al., Pharm Sci Tech 6 (2005) 618-625.

### Summary of the invention

The object of the present invention was to provide an approach to improved ezetimibe formulations.

In one aspect, the present invention provides a formulation of ezetimibe, comprising ezetimibe in the form of primary particles and a hydrophilic excipient in particulate form having ezetimibe particles adsorbed to.

According to another aspect, the present invention provides a formulation of ezetimibe, comprising ezetimibe combined with at least two types of hydrophilic excipients, wherein one type of hydrophilic excipient is starch and the other type of hydrophilic excipient is selected from saccharides including mono-saccharides, disaccharides and cellulose substances.

According to still another aspect, the present invention provides a formulation of ezetimibe comprising ezetimibe, granulated in admixture with up to 8 wt.-%, preferably up to 6 wt.-% and more preferably up to 4 wt.-% *),* for example 0.5 to 4 wt.-% of water or an aqueous liquid, or with up to 10 wt.%, preferably with 1 to below 10 wt.% of nonaqueous excipient selected from liquid substances.

According to yet another aspect, the present invention provides a formulation of ezetimibe comprising ezetimibe, admixed in solid solution with a solid solution agent.

According to a particular embodiment of the present invention, a process for producing a formulation of ezetimibe uses dry granulation in the absence of granulation liquid or in the presence of limited amount of water or aqueous liquid of up to 8 wt.-%, or in the presence of limited amount of non-aqueous liquid of up to 10 wt.-%, or uses solid solution formation of ezetimibe.

According to a further aspect, the present invention provides a pharmaceutical formulation comprising a formulation of ezetimibe according to anyone of the above aspects, or comprising a formulation of ezetimibe obtained by the aforementioned particular production process.

A further aspect of the invention is the use of any formulation according to the above aspects or the above pharmaceutical formulation for the prophylaxis or treatment of cholesterol-associated diseases, or a method of treatment by administering a cholesterol-decreasing amount of the formulation or the ezetimibe present as the active agent therein.

### Description of the invention, and of advantages and preferred embodiments thereof

The aspects, advantageous features and preferred embodiments of the present invention, respectively alone or in combination, contribute to provide improved ezetimibe formulations with a view of a good balance between free-flowing, cohesive powder and compression characteristics combined with yielding formulations of ezetimibe, and finally pharmaceutical formulations containing the same, enabling good chemical and physical stability of ezetimibe as well as good dissolution properties and acceptable levels of hardness when formulated in single unit dosage forms like tablets.

Further description is set forth in the following, referring to preferred embodiments and advantageous features, characteristics and effects, which are provided for illustrated purposes without an intention to limit the scope of the invention.

The ezetimibe substance is low water-soluble, low permeable, incompatible with most of the excipients usually used in drug formulation, presents stability problems, and is not inherently compressible. Consequently, there is a need to provide a free-flowing and cohesive ezetimibe composition capable of being formulated into dosage forms, especially when formulated at low strengths such as e.g. 10 mg doses or even only 5 mg doses of ezetimibe per unit dosage form, with an acceptable dissolution profile.

Despite of such constraints, it has been surprisingly found according to the invention that formulations of ezetimibe can be provided in a more robust and economical way and can display a good balance of properties including chemical and physical stability, free-flowing characteristics and compressibility as well as acceptable degrees of hardness and particularly acceptable to good dissolution profiles, when attention is paid, alternatively or optionally in combination, to (i) the existence of ezetimibe in the form of primary particles, which when merely blended, without co-milling, with a suitable hydrophilic excipient in particulate form are allowed, at a satisfactory percentage of primary particle status, to be adsorbed on the surface of particles of the hydrophilic excipient; (ii) a selection of specific types of excipients; and/or (iii) a careful control of how the formulation of ezetimibe is obtained. The aforementioned beneficial effects are more enhanced, if two or all of the afore-mentioned features (i), (ii) and (iii) are observed in combination.

According to a preferred embodiment, the ezetimibe for the purpose of blending and mixing with excipients, is provided in the form of primary particles in the ezetimibe formulation, preferably having a particle size d(0.9) of ≤ about 40 µm, more preferably ≤ about 25 µm, more preferably ≤ about 18 µm, and particularly at about 10 µm of, e.g., in a range of between 8.5 µm and 12.5 µm. Especially with a view to high dissolution rates, it has been found significant to ensure a physically stable form of primary particles of ezetimibe, and to reduce or preferably minimize the tendency towards formation of agglomerates. Combining particulate ezetimibe with a suitable hydrophilic excipient in particulate form allowed them adsorb onto the surface of the particulate hydrophilic excipient, thereby controlling and preferably minimizing the generation of ezetimibe agglomeration during processing and in both the formulation and the pharmaceutical formulation yielded thereby. In accordance with a preferred embodiment, it is possible according to the present invention to limit the proportion of agglomerated particles relative to the total of primary and agglomerated particles of ezetimibe to be below 20 %, preferably 10% or below and more preferably 5% or below (% indications denote number-%). The term "agglomerated particles" used herein typically means ezetimibe particles having a mean particle size of 20 µm or larger.
In order to achieve the significant advantages and effects, it is not only unnecessary but preferred to omit co-milling with other components or excipients of the formulation, rather to provide ezetimibe in the form of primary particles by being powdered, milled, peg-milled or micronized separately to have *per se* the desired particle size as such. This allows a control of adsorbing ezetimibe particles, preferably in the form of primary particles at relatively high rate, to the surface of the particulate hydrophilic excipient by blending. In particular, it allows a proper control and ratio between particle sizes of both ezetimibe and hydrophilic excipient, on whose surface the ezetimibe primary particles shall be covered by adsorption. It appears that ezetimibe in association with the particulate hydrophilic excipient by adsorption enables a better compatibility with further optional excipients, which then can be added at a higher proportion to further assist in achieving overall characteristics. Further, a tendency of chemical and/or physical changing of ezetimibe is reduced.

Preferably, it has been found beneficial to use the aforementioned relatively small primary ezetimibe particles - with possible limitation of agglomerated particles - for covering a relatively larger excipient particle by adsorption thereon. Suitable particle sizes of the hydrophilic excipient, onto which surface the ezetimibe particles are adsorbed to, is a range of from 10 to 400 µm (determined as mean particle size).

The appropriate status of the ezetimibe particles being adsorbed to the hydrophilic particulate excipient can be observed by suitable means, for example by means of scanning electron microscopy (SEM). Accordingly, the status of a surface of the particulate hydrophilic excipient being covered by small particles, preferably at a high ratio of primary particles of ezetimibe, is readily visible. The proportion of agglomerated particles relative to the total of particle forms can be determined by such suitable means also.

The hydrophilic excipient is suitably selected from the group consisting of saccharides, including polysaccharides and in particular mono- or disaccharides. The preferable selection among a saccharide for the hydrophilic excipient is to enhancing the adsorption state, and to maintainwe a high proportion of primary particles on the excipient particle surface. Preferred examples of hydrophilic excipients of saccharide type include particulate mono- or di-saccharides, including but not limited to lactose, isomalt, mannitol, sorbitol, glucose, fructose, lactitol, maltose, preferably mannitol, lactose and isomalt, and more preferably lactose and isomalt, respectively alone or in combination. Preferred polysaccharides include starch and cellulose substances, wherein preferred cellulose substances include microcrystalline cellulose, ethylcellulose, methylcellulose, hydroxypropyl cellulose, carboxymethyl cellulose and hydroxypropyl methyl cellulose, and crosslinked forms as well as combinations thereof.

According to a further preferred embodiment, the particulate hydrophilic excipient having said ezetimibe particles adsorbed thereto is intermixed with a binder. Binder as a matrix is able to further stabilize and enhance the adsorption status between particles of ezetimibe and hydrophilic excipient. Some of the aforementioned polysaccharides, onto whose surface ezetimibe particles can be adsorbed, can act at the same time as binders. Suitable binders include, but are not limited to microcrystalline cellulose, starch, calcium or sodium carboxymethylcellulose, cellulose, silicified microcrystalline cellulose, cellulose acetate, colloidal silicon dioxide, dextranes, dextrin, ethylcellulose, hydroxyethyl cellulose, hydroxyethylmethyl cellulose, hydroxypropyl cellulose, hypromellose, maltodextrin, methylcellulose, polydextrose, pregelatinized starch, zein, polyethylene glycol, polyvinylpyrrolidone, preferably starch, polyethylene glycol, preferably solid PEG such as PEG-4000 or PEG-6000, polyvinylalcohol (PVA) and polyvinylpyrrolidone (PVP).

The formulation with the ezetimibe particles - preferably in the form of primary particles as the main proportion with respect to all present ezetimibe particle forms - being adsorbed to the surface of the hydrophilic particulate excipient can be suitable obtained by blending both components, optionally concurrently together with further excipients. As a further option, further excipients, partly or completely, may be mixed after blending the ezetimibe and hydrophilic excipient particles. A good equipment for blending is a high shear mixer, assisting in a proper status of the ezetimibe particles being adsorbed to the particulate hydrophilic excipient, and allowing further excipients to form a matrix or network structure, preferably when a polysaccharide substance is used as a further excipient.

Independent to the aforementioned preferred embodiment of achieving a beneficial status of ezetimibe particles being adsorbed to the surface of a particulate hydrophilic excipient, the formulation of ezetimibe according to another embodiment of the present invention can be improved by selection of certain types of hydrophilic excipients. It is however noted that the present embodiment can be combined with the aforementioned preferred embodiment, which will particularly enhance desirable characteristics such as solubility, dissolution rate and profile. According to the present preferred embodiment, ezetimibe is combined with at least two types of hydrophilic excipients, wherein one type of hydrophilic excipient is starch and the other type of hydrophilic excipient is a saccharide selected from mono-saccharides, di-saccharides and cellulose substances. Although not necessarily, the two types of hydrophilic excipients are suitably present in the form of particles.
It has been surprisingly found that when ezetimibe as such, i.e. preferably without being co-milled with other components and without other active ingredients, is combined with both starch and suitably selected further saccharide, and especially combining with starch and mono- and/or di-saccharides, compatibility among the excipients and especially with the optional inclusion of further excipients is enhanced even at relatively low dosage percentages (corresponding to a high proportion of total excipients), tendency of chemical and/or physical changing of ezetimibe is reduced, and at the same time an excellent balance of characteristics including tablet hardness and good dissolution properties can be achieved. Thus, the combination of ezetimibe with both starch and further saccharide (in particular mono- or di-saccharide) not only favours compatibility of such excipients with ezetimibe and significantly further assists in limiting the generation of agglomerated ezetimibe particles, but additionally allows to add further excipients, which alone would have compatibility problems with ezetimibe but which on the other hand can additionally contribute to further beneficial effects. It is for example possible to add preferred choices of surfactants such as Tween, and to add preferred choices of binders such as polyethylene glycol, preferably solid polyethylene glycol, such as PEG-4000 or PG-6000, polyvinylpyrrolidone (Povidone; PVP) and/or polyvinyl alcohol (PVA).

Combined with a matrix or network formed by starch, the mono- and/or di-saccharid is preferably selected from the group consisting of lactose, isomalt, mannitol, sorbitol, glucose, fructose, lactitol, maltose, preferably lactose and isomalt. Starch can also be suitably combined with cellulose-type polysaccharide substances, including especially microcrystalline cellulose, ethylcellulose, methylcellulose, hydroxypropyl cellulose, carboxymethyl cellulose and hydroxypropyl methyl cellulose, and crosslinked forms as well as combinations thereof.

Independently from the preferred embodiments described above, but optionally alternatively thereto or in combination therewith, a preferred embodiment of the present invention relates to the choice of appropriate processing technology. Combination with the abovementioned preferred embodiments provides for the advantage of freedom to choose among the various known formulation technologies, including for example wet granulation, dry granulation and others known to the person skilled in the art. An advantage resides that the formulations of ezetimibe disclosed herein even enables the use of economically preferably dry granulation, although it is more challenging than wet granulation due to inherent difficulties involved by features attributable to the ezetimibe active ingredient as described above.

On the other hand, if improvements of ezetimibe formulation shall be accomplished independently from the abovementioned preferred embodiments, the present invention provides further beneficial embodiments by carefully controlling how the formulation of ezetimibe is obtained. One particularly developed technology is the granulation in admixture with a limited amount of liquid. It has been surprisingly found that granulation in admixture with a limited amount of water or an aqueous liquid of up to 8 wt.-%, preferably up to 6 wt.-% and more preferably up to 4 wt.-%, for example 0.5-4 wt.-%, more specifically 1 to 4 wt.-% and particularly 2 to 3 wt.-%, or granulation in admixture with a limited amount of an non-aqueous excipient selected from liquid substances up to 10 wt.-%, for example from 1 to below 10 wt.-%, respectively relative to the total amount of formulation of ezetimibe subjected to granulation, and subsequently subjecting the blend to granulation without further granulation fluid, an ezetimibe formulation can be obtained where free-flow characteristics, cohesive characteristics and compressibility of the ezetimibe formulation is enhanced, and in addition good dissolution properties are displayed. Since, besides the limited amount of liquid excipient, otherwise dry conditions are set, a drying step becomes superfluous. This can be further assisted by addition of a binder capable of absorbing the limited amount of liquid excipient. Selection of solid excipients from solid polyethylene glycol, isomalt, microcristalline cellulose (e.g. Avicel), mannitol and lactose have been found to be particularly suited for dry granulation with limited amount of liquid.

In the present embodiment of adding limited amount of liquid excipient during granulation, either water alone or an aqueous liquid can be used. In the aqueous liquid, water can be mixed with water-miscible organic solvents, such as lower alcohols, alkyl ethers, and the like, for example methanol, ethanol, n- or iso-propanol, butanol, pentanol, hexanol, heptanol, or octanol. Alternatively or in combination with aqueous liquid, when using non-aqueous liquid substances for dry granulation assistance, the non-aqueous excipient can be selected from liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol. The preferred non-aqueous excipient is a liquid surfactant, preferably of non-ionic type, more preferably selected from the group of Tweens, sodium dodecylsulfate and poloxamers, more preferably selected from Tweens and particularly choosing Tween 80.

Another processing technology found to be particularly suited is by admixing ezetimibe in solid solution with a solid solution agent. This particular processing technology contributes to good dissolution properties, especially with respect to dissolution rate. The term "solid solution" used herein is a single phase existing in an admixture with ezetimibe. The solid solution may be limited to only the ezetimibe and the solid solution agent and optional further excipients solubilized in the solid solution, or it may be combined with one or more separate, unsolubilized or particulate, further optional excipient. The solid solution component can typically be ascribed as a single phase existing over a range of chemical compositions in which ezetimibe is dissolved or dispersed. Some minerals are able to tolerate a wide and varied chemistry, whereas others permit only limited chemical deviation from their ideal chemical formulae. In many cases, the extent of solid solution is a function of temperature, with solid solution being favoured at high temperatures, whereas unmixing and/or ordering being favoured at low temperatures.

In the embodiment for preparing solid solution of ezetimibe, solid solution agent, which is suitable selected from appropriate excipient forms that are solid *per se,* and liquid media are used for preparing solid solution in liquid. A suitable solid excipient to be used as solid solution agent can be selected from binder substances, preferably from the group consisting of colloidal silicon dioxide, polyvinylpyrrolidone, polyethylene glycol, and polyvinylalcohol (PVA). Most preferred is use of solid polyethylene glycol (PEG), in particular one falling in the molecular weight in the range of 4000 to 20000 such as PEG-4000 or PEG-6000, and polyvinylpyrrolidone (PVP), optionally also polyvinylalcohol (PVA). Two or more of the aforementioned solid solution agents, especially when using PEG and PVP, optionally further including PVA, can be used in combination to further enhance effects.

Suitable liquid media for preparing the solid solution include organic solvents such as lower alcohols, for example methanol, ethanol, n- or iso-propanol, butanol, pentanol, hexanol, heptanol, or octanol, and mixtures of organic solvents with water. Preferably ethanol and aqueous ethanol solutions are used. After a solution of ezetimibe and the solid solution agent has been prepared, optionally including a heating treatment for making homogenous or evenly distributed solution, the liquid medium component used for preparing solid solution in liquid is evaporated. Evaporation is performed more effectively at elevated temperature, for example at range of 40 to 50°C. This is moist efficiently done during a fluid bed top spray granulation procedure.

Solid solution agents which concurrently act as binders will also improve compatibility with additionally included, optionally particulate excipients. Optional additional excipients may be included by solid solution formation as well, or they are included after solid solution formation of ezetimibe by suitable methods, such as fluid bed granulation techniques.

For the aspects and preferred embodiments described above, the following features are of further particular significance.

When processing ezetimibe by a technology as described above, ezetimibe can suitably be present in particulate form, although not being necessarily limited thereto. In certain embodiments, other forms such as a dissolved state is more suitable, for example when choosing solid solution formulations of ezetimibe.

Since each of the aforementioned aspects and preferred embodiments, either alternatively or in combination, contribute to an improved compatibility between excipients, there is a beneficial freedom of choice among a larger variability of excipients to be used depending of the desired functionality to be achieved, and thus enabling beneficial effects attributable to the selected excipient. Thus, intended for the final pharmaceutical formulation, e.g. existing as single dosage form such as tablet, several ingredients can be used besides ezetimibe and optionally further active pharmaceutical ingredients. For example, excipient ingredients may be used to improve mechanic characteristics, such as bulk, improved flow, better compressibility, improved disintegration characteristics, hardness, taste, and tablet performance, flavoring, or enhanced appearance. Many types and examples of excipients can be used to accomplish such functionalities, including but not limited to binders, fillers, lubricants, disintegrants, surfactants, coloring agents, diluents, antiadhesives, glidants, alone or in combination. Further useful additives may include, alone or in combination, buffer agents, anti-oxidants, colorants, stabilizers, fillers, plasticizers, emulsifiers, preservatives, viscosity-modifying agents, or passifiers, flavouring agents, without being limited thereto.

The balance of properties especially including dissolution properties and chemical and physical stability of the ezetimibe active component is at good levels when the excipient is selected from specially adapted solid excipients, including for example those selected from the group consisting of solid polyethyleneglycol (PEG; Macrogol), preferably PEGs having molecular weights in the range of 4000 to 20000; polyvinyl pyrrolidone (PVP) or its crosslinked form crospovidone; silicon dioxide, preferably fumed silica or colloidal silicon dioxide; moni-/di-/polysaccharides including sugar esters and polyhydroxy-sugars, preferably mannitol; lactose, isomalt, lactitol; sorbitol, glucose, fructose, maltose, preferred polysaccharides of dextranes, dextrin, maltodextrin, and dextrose, more preferably starch and celluloses, methylcellulose, cellulose acetate, hydroxyethylcellulose (HEC), hydroxypropylcellulose (HPC), hydroxypropyl methylcellulose (HPMC), HPMC phthalate, polydextrose, cyclodextrins and carbomers, microcrystalline cellulose (MCC), silicified microcrystalline cellulose, powdered cellulose, carboxymethylcellulose (calcium or sodium), hypromellose, pregelatinized starch, zein; buffer salts, preferably anhydrous mono-, di- or tribasic phosphate, more preferably calcium phosphate, calcium sulfate and calcium silicate, magnesium carbonate; hydrophilic, anionic, cationic, zwitterionic and non-ionic surfactants and lipohilic additives such as mono-, di- or triglycerides, polyethoxylated fatty acids, fatty acid esters and oils, e.g. sodium oleate, sodium lauryl sulfate and magnesium stearate and polyglycolized glycerides; talc; polypeptides such as gelatine; and mixtures thereof; without being limited thereto. A single dosage pharmaceutical formulation as an embodiment of the present invention may further comprise a cover film or shell, which according to a preferred embodiment is non-hygroscopic as well. In further preferred embodiments, bi- or multifunctional excipients can be used, for example, colloidal silicon dioxide (CSD) admixed with micro-crystalline cellulose (MCC), for example Prosolv™. Excipients used preferably include at least one or more of the group consisting of polyethylene glycol, isomalt, microcristalline cellulose, mannitol and lactose, preferably comprising at least starch, lactose, isomalt, PEG, PVP and Tween, optionally also croscarmellose sodium.

When desired, the present invention allows to take advantage of a low weight percentage, or low absolute dose per dosage unit, of active principle of ezetimibe, especially where ezetimibe is the sole active ingredient. This concept in turn means a high weight percentage or high absolute amount of appropriate excipients, with the possibility of selecting useful and effective amounts of excipient depending on the desired magnitude of the attributable effect, including especially free-flowing, cohesive and compressibility characteristics during processing, dissolution property despite of very low solubility of ezetimibe alone (typically being below 0.01 mg/ml in water), and physical and/or chemical stability in spite of ezetimibe being prone to stability challenges. Thus, in certain beneficial embodiments, the balance of the ezetimibe formulation may formed, besides ezetimibe, only by excipients of appropriate choice, only optionally further including a second or third, different active ingredient. This applies for example to cases wherein the content of ezetimibe is at a dose of up to 25 mg, or up to 20 mg per dosage form, including e.g. a standard level of about 10 mg or about 5 mg. Percentage level of ezetimibe in the final pharmaceutical formulation can be suitably selected as desired, for example at the proportion below 50 wt.-%, more suitably below 25 wt.-%, notably at a level of around 10 wt.-% or below, or at a level of around 5 wt.-%, respectively relative to the total weight of the pharmaceutical formulation.

The pharmaceutical formulation finally used for medical purposes comprises any one of the formulations of ezetimibe described in the various aspects, preferred embodiments and particular features described above. In this connection, it is noted that various technologies can be used and selected depending on the magnitude of finally desired predominant effect. In particular, technologies can be preferably selected from the group consisting of wet granulation, dry granulation either in the absence of granulation liquid or in the limited presence of aqueous or non-aqueous excipients suitable for dry granulation, fluid bed granulation or direct compression. Processing may suitably include one or more steps of briquetting or slugging, sieving, milling, blending, tabletting and compression. It may also include direct compression. As described above, formulations disclosed herein enable advantageous and particularly preferred technologies, including classical dry granulation without using granulation liquid, or modified dry granulation working with a limited amount of aqueous (up to 8 wt.-%) or non-aqueous liquid (up to 10 wt.-%) as a kind of moisture-activation; and solid solution formation and/or fluid bed granulation, which respectively makes the process of forming single unit dosage forms such as tablets containing ezetimibe more robust and economical, while enabling suitable dissolution profiles.

Despite the advantages of the dry granulation, such as reduced processing time and cost, wet granulation is in general and widely used in the industry to prepare solid dosage forms. Wet granulation is often preferred over dry granulation and direct compression because wet granulation has a greater chance of overcoming any problems associated with the physical characteristics of various ingredients - active or as excipient - in the formulation, with a view of providing material which has the required flow and cohesive properties necessary to obtain an acceptable solid dosage form. In general, while segregation could be a potential problem with direct compression and dry granulation, sizes and shapes of particles comprising granulate to be compressed are more easy controlled through wet granulation process. In addition, when a dry solid is wet granulated the binder "glues" particles together, so that they agglomerated into spherical granules.

In spite of the apparent ease of processing afforded by wet granulation, many manufacturers would welcome the opportunity to dry granulation tablets, especially in cases when low dose of ezetimibe is desired. It is a benefit of the present invention to meet this need in the industry for techniques and pharmaceutical formulations which will allow manufacturers to prepare low dose ezetimibe tablets by dry granulation, which will avoid the time and expense of wet granulation, as well as higher solubility of active principle in final formulation.

The pharmaceutical formulation according to the present invention allows to achieve a beneficial dissolution profile by releasing ezetimibe at a rate of higher than 30 %, preferably higher than 40 %, and even as high as at least 60 %, within 30 minutes, relative to the original amount of eprosartan in the formulation, tested using USP apparatus 2, placing the formulations in 1000 ml 0.1 M hydrochloric acid at 37 ± 0.5°C with paddle speed of 50 rpm.

In dry granulation methods, the powder particles are associated under high pressure. The components of the formulation are compressed in the dry stage. If the sufficient bonding strength can not be achieved by compression alone, a binder is added, also in the dry state. The initial compression stage can take place by one of two methods. The first uses a conventional tablet press, a process often referred to as slugging or briquetting. Since the components of the formulation will not have the necessary attributes for producing tablets with acceptable characteristics (hardness, friability, weight, shape, disintegration, dissolution), the tablets produced at this stage (the slugs or briquettes) will not be of acceptable quality, especially as regards to appearance and weight uniformity. Therefore, the briquettes are then broken down to form a granular product, which after sieving can then be compressed again to give satisfactory tablets. It was proved that the ease of compressibility of the formulation at the second compression was inversely proportional to the pressure used at the briquetting stage, implying that briquetting at high pressure should be avoided.

A unique modification of dry granulation process was found to be particularly suited for ezetimibe formulations. Accordingly, in our work we developed granulation of ezetimibe powder with limited amount of water, aqueous liquid or non-aqueous liquid and further optional excipients. This modification is denoted Moisture Activated Dry Granulation (MADG) in case of using limited amount of water, or Non-Aqueous Moisture Activated Dry Granulation (NAMADG) in case of using limited amount of non-aqueous liquid excipient such as surfactant. As a further, alternative effective for achieving good dissolution profiles of ezetimibe, both limited amount of water and limited amount of non-aqueous liquid excipient such as surfactant are useful, named here Double Moisture Activated Dry Granulation (DMADG). In case of DMADG, ratio of water to non-aqueous liquid excipient is preferably from 1:4 to 4:1. Such particularly developed modifications of dry granulation turned out to be very suitable for ezetimibe having poor flowability and insufficient compression properties (especially if higher drug doses are desired), and having poor solubility (still relevant for low dose formulations). An adjustable amount of binder capable of absorbing the limited amount of liquid excipient can be added. The ingredients can be mixed, optionally in a high speed mixer, within a suitable wet massing time, which means the time of mixing after the liquid excipient has been added. The preferred wet massing time is up to 10 minutes, more preferably 2 to 5 minutes. Traditional drying step can be beneficially omitted.

The fluid bed granulation process involves suspending particulates in an air stream and spraying a liquid from the top down onto the fluidized bed. Particles in the path of the spray get slightly wetted and become tacky. The tacky particles collide with other particles and adhere to them to form a granule. There are two different modes of fluid bed granulating: Dry stage and Wet stage. In Dry stage granulation, the particles only require a slight wetting to become tacky and stick to each other. The granulating solution is applied at a rate less than or equal to the evaporation rate. Thus the particles remain "dry" through the entire process. In Wet stage granulation, the particles require significant wetting before they become tacky enough to stick to each other. The granulating solution is applied at a rate higher than the evaporation rate until the particles build up enough moisture to granulate. The characteristics of the particles when wet and the type of granulating solution being used will determine which mode of granulating is most appropriate. While Dry stage is more common, Wet stage granulating allows for denser products. Fluid bed granulation is commonly used to develop porosity and effective surface area, which consequently gives higher solubility of active principle.

The following examples are merely illustrative of the present invention and they should not be considered as limiting the scope of the invention in any way, as these examples and modifications as well as equivalents thereof will become apparent to those versed in the art in the light of the present disclosure and the accompanying claims. In the drawings:
- Fig. 1: shows dissolution profiles of different combinations of ezetimibe and excipients after blending, using USP dissolution test apparatus 2 at 0.01 M HCl and 0.25% SDS (900 ml);
- Fig. 2: shows a SEM of ezetimibe as such, having a d (0.9) of about 10 µm;
- Fig. 3: shows a SEM of ezetimibe and starch after blending;
- Fig. 4: shows a SEM of ezetimibe and isomalt after blending;
- Fig. 5: shows a SEM of ezetimibe and lactose after blending;
- Fig. 6: shows a SEM of ezetimibe with lactose and starch (Fig. 6a) and isomalt and starch (Fig. 6b) respectively after blending;
- Fig. 7: shows the influence of various technologies for processing ezetimibe, and the use of various functional excipients (900 ml);
- Fig. 8: shows further influence factors of technologies as well as the use of specific functional excipients (500 ml);
- Fig. 9: shows a SEM of ezetimibe in solid solution with polyethylene glycol (Fig. 9a) and with polyvinylpyrrolidone (Fig. 9b), respectively;
- Fig. 10: shows the dissolution profile of ezetimibe tablets produced in Example 10 (500 ml); and
- Fig. 11: shows dissolution profiles of different combinations of ezetimibe and excipients after blending, activating, and tableting, using USP dissolution test apparatus 2 at 0.01 M HCl and 0.25% SDS (500 ml);

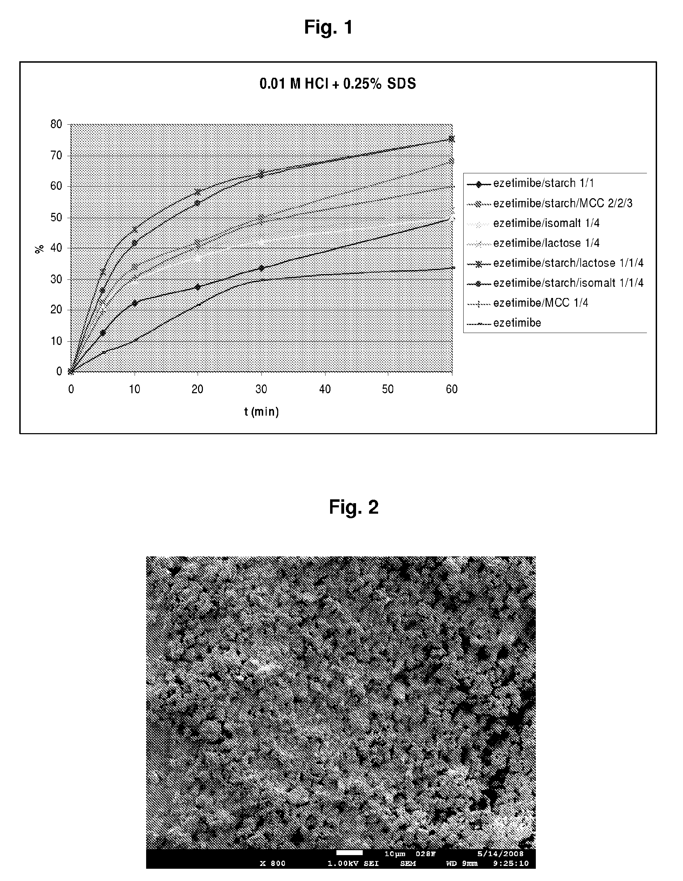

As shown as in Fig. 1, the selection of excipient to be combined with ezetimibe significantly affects the dissolution profile of ezetimibe. A combination of ezetimibe in particulate form with starch and saccharide lactose or isomalt remarkably enhances dissolution rate, compared to other combinations defined by lower compatibility.

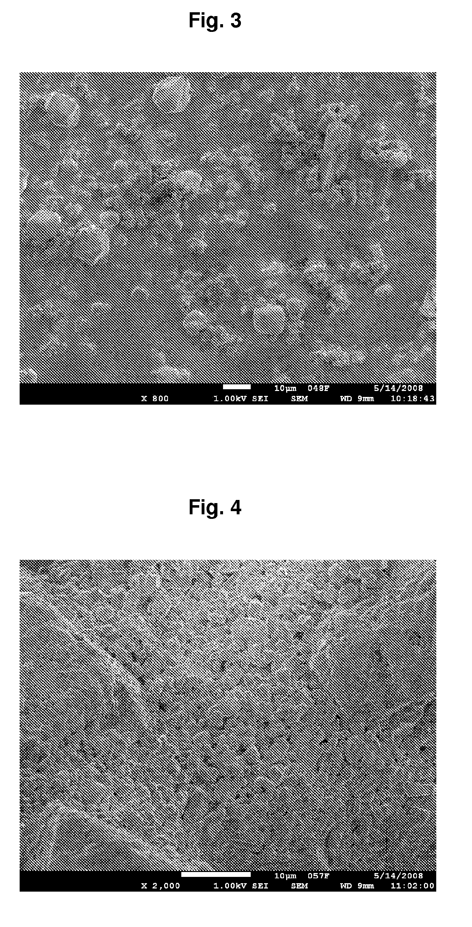

Fig. 2 shows the particulate structure and nature of ezetimibe as such, i.e. absent from other active ingredients and without being co-milled with excipients for reference, whereas Figs. 3 to 6 show the significance of forms of ezetimibe particles being adsorbed to various selected hydrophilic excipients being present themselves in a particulate form.

As shown in Fig. 3, blending of ezetimibe with starch results in some agglomeration of ezetimibe. However, when ezetimibe is blended with isomalt, the surface of isomalt is substantially covered with small particles of ezetimibe thus favoring the existence of primary particles and controlling and preferably minimizing the tendency of formation of agglomerated particles, as shown in Fig. 4. Compared with the situation of starch shown in Fig. 3, a substantially higher amount of ezetimibe particles are present on the surface of starch.

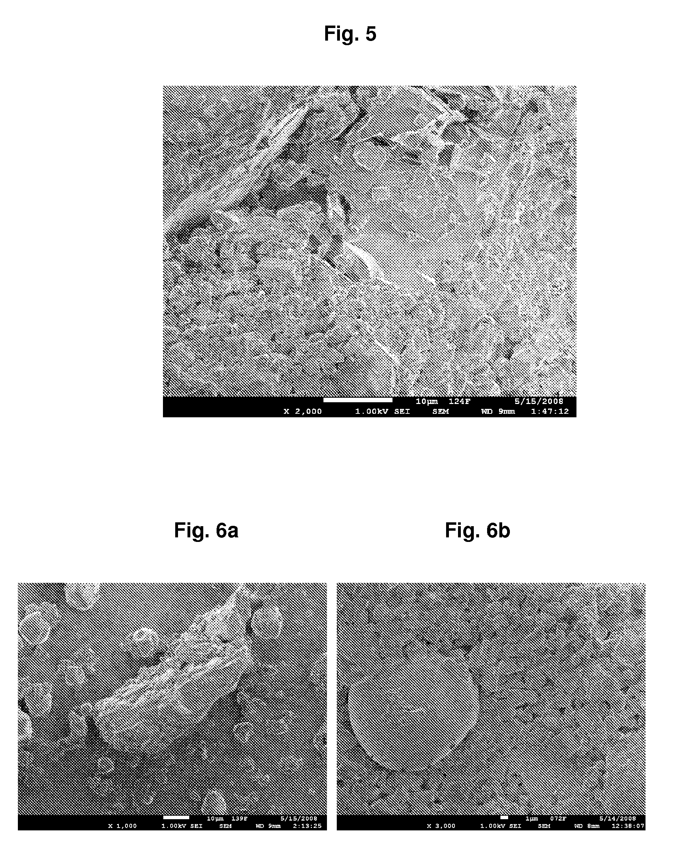

Likewise, Fig. 5 shows the result of blending ezetimibe with lactose, wherein the surface of lactose is again covered substantially by small particles of ezetimibe.

Fig. 6 shows the result of a beneficial combination of ezetimibe with starch and saccharide, presently illustrated by the specific choice of lactose or isomalt as preferred disaccharide. As shown, lactose and/or isomalt respectively combined with starch as excipient components are especially beneficial by contributing to the effects of covering the surface of lactose by small particles of ezetimibe, while isomalt is covered by starch with ezetimibe and by ezetimibe alone.

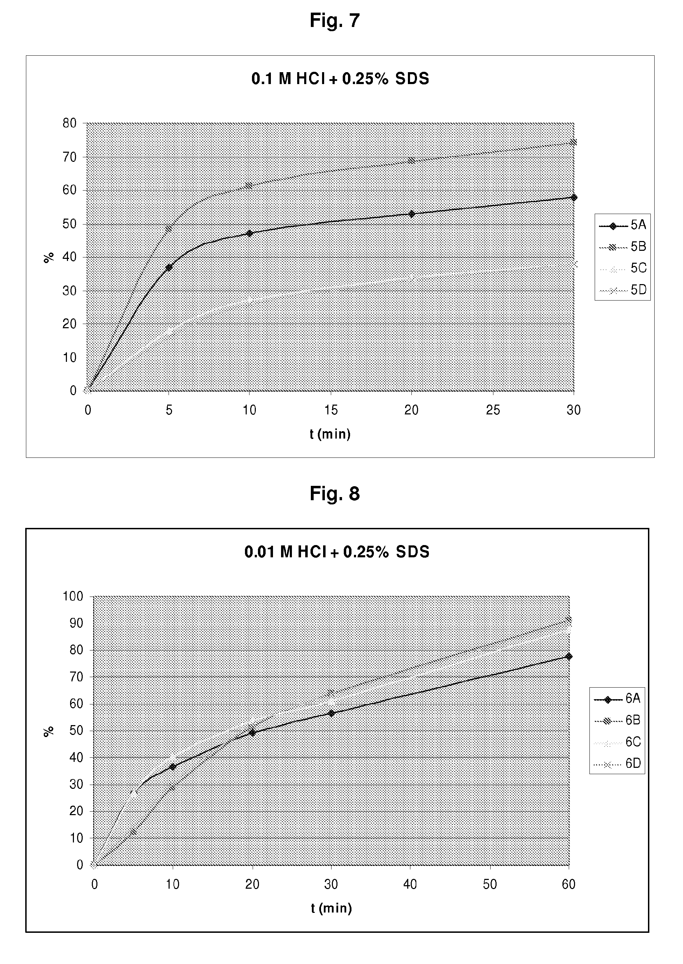

Fig. 7 shows the influence and significance of various technologies on dissolution profiles of ezetimibe formulations. As shown, the inclusion of surfactant, in particular Tween, enhances dissolution rate, especially in combination with the proper choice of techniques using either wet granulation, or dry granulation performed in the presence of limited amount of liquid excipient, i.e. limited amount of surfactant (Tween) and/or water. With a view of avoiding more expensive and economically inferior wet granulation, it becomes apparent that conditions of dry granulation with a limited amount of liquid excipient establishes a robust and economically beneficial technology combined with acceptable dissolution properties.

Fig. 8 shows further results of the influence and significance of technologies and selection of relevant excipients, again with a view of circumventing wet granulation. It shows that acceptable results can be obtained by technologies like fluid bed granulation and dry granulation in the presence of limited amount of liquid excipient such as surfactant and/or water. Data to elucidate effects of various technologies and excipients can be further depicted from Table 7 below.

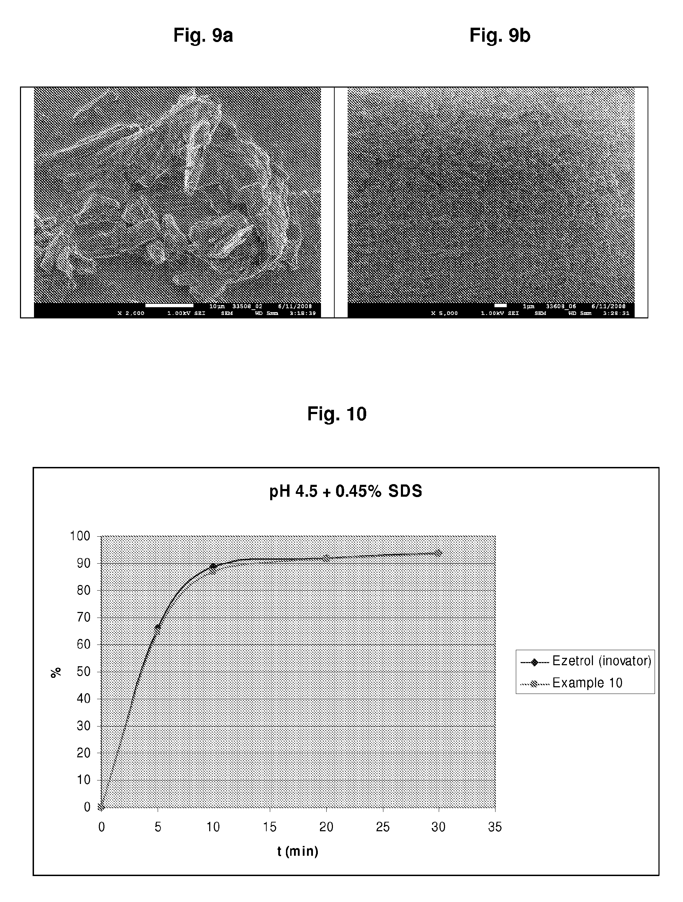

Fig. 9 shows suitable physical status of ezetimibe when present in solid solution with suitable solid solution agents, illustrated here with a selection of the excipients polyethylene glycol 4000 (Fig. 9a) and polyvinylpyrrolidon (PVP K-25; Fig. 9b). As has been shown in dissolution tests, PEG and PVP display positive influence on an increasing solubility of ezetimibe present in solid solution. Therefore, solid solution provides a useful alternative to wet granulation and other technologies. Furthermore, further tests have shown that suitable binders can enhance binding properties between particles; for example, polyvinyl alcohol (PVA) has been found to act as a suitable binder to be preferably combined with ezetimibe.

Fig. 10 shows excellent results on dissolution profile of ezetimibe tablets which have been obtained by dry granulation technology with limited amount of non-aqueous liquid excipient (Tween 80). In further investigations with this type of technology, it was found that non-aqueous liquid excipient should be in a content range of up to 10wt.-%, for example 1 to 10 wt.-% of the whole amount of formulation subjected to granulation. Excellent results of tablets comprising Tween as the selected liquid excipient for activated dry granulation, with excellent dissolution properties and disintegration properties (≤ 5 min) was achieved with 2.5 wt.-% of Tween 80.

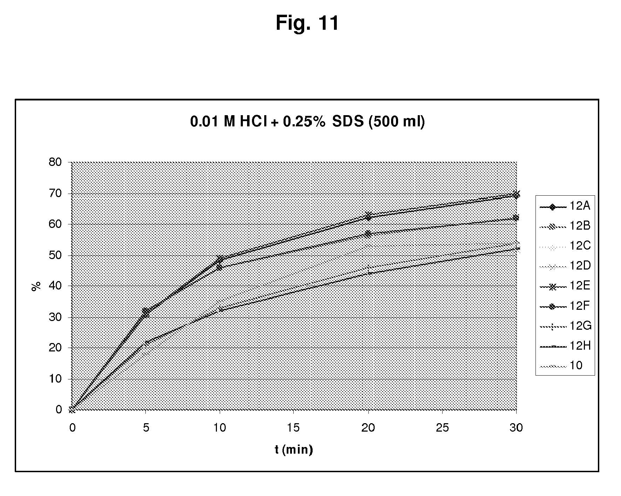

Fig. 11 shows excellent results on dissolution profile of ezetimibe tablets which have been obtained by dry granulation technology with limited amount water, with or without limited amount of non-aqueous liquid excipient (Tween 80). Further, positive effect of various excipients in blending with ezetimibe become apparent, especially when blended with particulate saccharide including lactose, which is preferably a spray dried one (SD type), or isomalt, especially since blending is respectively used with starch.

Observations which can be inferred from the results conducted with variations of formulation samples described in different examples include:
Preferred embodiments of the invention can provide an ezetimibe formulation in the form of a free-flowing, cohesive powder and tabletting granulate, capable of being compressed into a tablet of small amount (e.g. tablet size of 20 to 250 mg, preferably 50 to 150 mg, notably a standard size of 100 mg).
Ezetimibe formulations disclosed herein enables to provide a compressed ezetimibe tablet in unit dosage form having an acceptable dissolution profiles as well as acceptable degrees of hardness (50-100 N) and resistance to chipping, during a short disintegration time.
Ezetimibe formulations disclosed herein further promotes to provide a process for preparing a compressed ezetimibe tablet by dry granulation, moisture activated dry granulation (MADG), non-aqueous moisture activated dry granulation (NAMADG), double moisture activated dry granulation (DMADG), wet granulation, or fluid bed granulation in unit dosage form, without physical or chemical change of active principle.

Positive influence of mixing ezetimibe with different combinations of starch and saccharides, in particular starch+lactose and/or starch+isomalt, on dissolution profile was confirmed.

Surfactants like Tweens and poloxamers showed significant changes in dissolution profile of ezetimibe dependent on used percentage and technology.

Particuar examples also demonstrated higher solubility of solid solution formulations of ezetimibe with different amount of suitable binder substances, using as preferred examples PVP (povidone), PEG-4000 or PEG-6000 (polyethylene glycol), and PVA (polyvinyl alcohol) respectively in liquid medium such as ethanol which is subsequently evaporated.

### Examples

### Examples 1 A-1 E:

Based on the use of micronized active principle, firstly direct compression as the manufacturing process was investigated. Mixture of active substance and all other excipients shown in Table 1 below, except magnesium stearate, was homogenized and sieved (0.7 mm). Then, magnesium stearate was added and homogeneously mixed and tried to compress into tablets (mass 100 mg). Owing to very low solubility of ezetimibe (below 0.01 mg/mL in water) and low percentage of active principle (10%) in comparison to excipients, direct compression with most commonly used excipients in different combinations did not bring acceptable dissolution profiles (in 30 min less then 80% of API released in all investigated dissolution juices).

**Table 1. Composition of Ezetimibe 10 mg tablets (direct compression).**

| Constituent | **Examples** | | | | | Function |
|---|---|---|---|---|---|---|
| | **1A** | **1B** | **1C** | **1D** | **1E** | |
| Ezetimibe | 10.00 mg (43 µm) | 10.00 mg (43 µm) | 10.00 mg (17 µm) | 10.00 mg (17 µm)* | 10.00 mg (17 µm) | Active |
| Ludipress^{™} | 89.50 mg | / | 89.50 mg | 89.50 mg | / | Filler, binder |
| Prosolv^{™} (Silicified MCC) | / | 89.50 mg | / | / | / | Filler |
| Lactose spray dried | / | / | / | / | 87.00 mg | Filler |
| Croscarmellose sodium | / | / | / | / | 2.50 | Disintegrant |
| Magnesium stearate **anhydrous form of ezetimibe* particle size of ezetimibe in Table 1 and Tables below, where indicated, is d(0.9) | 0.50 mg | 0.50 mg | 0.50 mg | 0.50 mg | 0.50 mg | Lubricant |

### Example 2:

Further investigation was done by dry granulation process including briquetting step, with ezetimibe and excipients generally listed in Table 2, namely specifically 89.0% of lactose (70-100 mesh or SD), isomalt, avicel PH 101 (MCC), lactose / starch (79/10), isomalt / starch (79/10), avicel PH101/starch (79/10), lactose / starch / avicel PH 101 (55/10/24), and isomalt / starch / avicel PH 101 (55/10/24). In all cases, 1% of magnesium stearate was added. The ingredients (API and excipients) were weighted and blended in laboratory blender purpose for 5 min. Subsequently, mixture of powders was used for compressing into briquettes using a rotary tablet press in a controlled environment. After that, briquettes were grinding twice through 2.0 mm and then 1.2 mm sieve. Afterwards, external excipient (magnesium stearate) was screened through a 0.3 mm screen, added to granulate, and mixed for 1 minute. The resulting final blend was compressed into tablets using a rotary tablet press in a controlled environment. Tablets were compressed at a compression weight of 100 mg using capsule-shaped punches.

Ingredients selected for further ezetimibe tablets investigation are shown in Table 2.

**Table 2. Function and approximate amount of chosen excipients.**

| Excipient | Approximate amount | Function |
|---|---|---|
| | (%) | |
| Lactose monohydrate 70-100 mesh | Up to 89.50 | Diluent |
| Lactose DCL 14^{™} | Up to 89.50 | Diluent |
| Lactose DCL 21^{™} | Up to 89.50 | Diluent |
| Maize starch | Up to 10.00 | Disintegrant |
| StarLac^{™} | Up to 75.00 | Diluent, Disintegrant |
| Crospovidone (Polyplasdon XL^{™}) | Up to 2.00 | Disintegrant |
| Croscarmellose sodium | Up to 8.00 | Disintegrant |
| Cellulose microcrystalline | Up to 89.50 | Filler, Binder |
| (Avicel PH 101^{™}) | | |
| Cellulose microcrystalline | Up to 89.50 | Filler, Binder |
| (Avicel PH 102^{™}) | | |
| Cellulose microcrystalline / mannitol | Up to 75.00 | Filler, Binder |
| (Avicel HFE 102^{™}) | | |
| Cellulose microcrystalline | Up to 25.00 | Filler, Binder |
| (Avicel PH 200 LM^{™}) | | |
| Prosolv^{™} (Silicified MCC) | Up to 89.50 | Filler, Binder |
| Macrogol 4000 (PEG-4000) | Up to 20.00 | Binder, Solid solution agent |
| Macrogol 6000 (PEG-6000) | Up to 20.00 | Binder, Solid solution agent |
| Isomalt (GalenlQ721^{™}) | Up to 89.50 | Binder, Diluent |
| Colloidal silicon dioxide (Aerosil 200^{™}) | Up to 1.50 | Glidant |
| Magnesium stearate | 0.25 - 2.00 | Lubricant |
| Texapon K 12^{™} (sodium dodecyl sulfate) | Up to 10.00 | Surfactant |
| Tween 80 (polysorbate) | Up to 5.00 | Surfactant |
| Tween 60 (polysorbate) | Up to 5.00 | Surfactant |
| Tween 20 (polysorbate) | Up to 5.00 | Surfactant |
| Pluronic F-68^{™} (poloxamer 188) | Up to 5.00 | Surfactant |
| Hypromellose (Pharmacoat 606) | Up to 2.50 | Binder |
| Klucel EXF^{™} (Hydroxypropylcellulose) | Up to 3.00 | Binder, Disintegrant |
| PVP K-25 (povidone) | Up to 20.00 | Binder, Solid solution agent |
| PVA (Polyvinyl alcohol) | Up to 20.00 | Solid solution agent |
| Calcium silicate | Up to 10.00 | Disintegrant, Porosity |
| | | agent |

Dissolution profiles from the different combination of ezetimibe and excipients after blending in high shear mixture (Mi-PRO; 500 rpm) are shown as in Fig. 1. Legends are as follows: lactose - lactose 70-100 mesh; starch - maize starch; isomalt - galenlQ721; MCC - Avicel PH 102. As shown as in Fig. 1, a combination of ezetimibe in particulate form with appropriate saccharide, notably with both starch and another saccharide, affects and partly remarkably enhances dissolution rate, compared to other combinations defined by lower compatibility. Starch combined with lactose, isomalt or microcristalline cellulose as the other saccharide is useful in terms of dissolution enhancement.

While in Fig. 2 the particulate structure and nature of ezetimibe alone is shown for reference, Figs. 3 to 6 show the significance of forms of ezetimibe particles being adsorbed to various selected hydrophilic excipients being present themselves in a particulate form.

Blending of ezetimibe with starch alone results in some agglomeration of ezetimibe (Fig. 3). Situation is improved when an ezetimibe is adsorbed onto and covers the surface of other blended hydrophilic saccharide excipient in particulate form, shown here with isomalt. Substantial amounts of ezetimibe particles, mainly in the form of small (and even primary particles) are present on the surface of particulate isomalt, and the tendency of ezetimibe particle agglomeration is limited (see e.g. Fig. 4).

Similar results on beneficial adherent association of a high number of small particles of ezetimibe are obtained when blending ezetimibe with another saccharide, lactose (cf. Fig. 5).

Further improvement is achieved by a combination of ezetimibe with starch and other saccharide, illustrated in Fig. 6 after blending of ezetimibe with a) lactose/starch and b) isomalt/starch. Surface of lactose is covered by small particles of ezetimibe while isomalt is covered by starch with ezetimibe.

Further in terms of dissolution testing results, level of ezetimibe's agglomeration in final formulation a significant affecting parameter. High percentage of agglomerates in tablets is detrimental to reach good dissolution profile. Blending of ezetimibe with appropriate amount of hydrophilic excipients such as saccharides, in particular isomalt and/or lactose, assists in covering ezetimibe in adherence to surfaces to thereby maintain a high ratio of small and especially primary particles. Further combination with network structure of suitable binders, in particular in combination with starch, is assumed to assist in braking down possible agglomerates to small ezetimibe particles.

### Examples 3A and 3B:

The next step was wet granulation with water or ethanol as granulation fluid in combination with some of the most promising excipients in previous Examples, which are depicted in Table 3.

**Table 3. Composition of Ezetimibe 10 mg tablets (wet granulation):**

| Constituent | **Examples** | | Function |
|---|---|---|---|
| | **3A** | **3B** | |
| Ezetimibe | 10.00 mg | 10.00 mg | Active |
| Cellulose microcrystalline (Avicel PH 101^{™}) | 12.00 mg | 12.00 mg | Filler, binder |
| Lactose 200 mesh | 72.00 mg | 72.00 mg | Filler |
| Texapon K 12^{™} (sodium dodecyl sulfate) Crospovidone (Polyplasdon XL^{™}) | 1.50 mg 1.50 mg | 1.50 mg 1.50 mg | Surfactant Disintegrant |
| Hypromellose (Pharmacoat 606^{™}) | 2.50 mg | 2.50 mg | Binder |
| Purified water | q.s. | / | Granulation fluid |
| Ethanol (96%) | / | q.s. | Granulation fluid |
| Magnesium stearat | 0.50 mg | 0.50 mg | Lubricant |

Using either purified water or ethanol as granulation fluid in wet granulation brings significant problems with uniformity of mass (RSD ≥ 2%) as well as agglomeration and segregation of ezetimibe.

### Examples 4A-4C, Examples 5A and 5B, and Examples 6A-6D:

Therefore, limitation of granulation liquid in a concept of moisture activated dry granulation (MADG) was used in next step of testing. Ingredients for MADG and variations are shown in Tables 4 to 6 for Examples 4 to 6, respectively.

**Table 4. Composition of Ezetimibe 10 mg tablets (MADG)**

| Constituent | **Examples** | | | Function |
|---|---|---|---|---|
| | **4A** | **4B** | **4C** | |
| Ezetimibe (17 µm) | 10.00 mg | 10.00 mg | 10.00 mg | Active |
| StarLac^{™} (lactose, starch) | 74.50 mg | / | / | Filler, disintegrant |
| galenlQ721^{™} (isomalt) | / | 74.50 mg | / | Filler |
| Avicel HFE-102^{™} (MCC, mannitol) | / | / | 74.50 mg | Filler |
| Crospovidone (Polyplasdon XL^{™}) | 1.00 mg | 1.00 mg | 1.00 mg | Disintegrant |
| Croscarmellose sodium | 6.00 mg | 6.00 mg | 6.00 mg | Disintegrant |
| Klucel EXF^{™} | 3.00 mg | 3.00 mg | 3.00 mg | Binder, disintegrant |
| (Hydroxypropylcellulose) | | | | |
| Tween 80 (polysorbate) | 2.50 mg | 2.50 mg | 2.50 mg | Surfactant |
| Purified water | 2.50 mg | 2.50 mg | 2.50 mg | Granulation fluid |
| Magnesium stearat | 0.50 mg | 0.50 mg | 0.50 mg | Lubricant |

**Table 5. Composition of Ezetimibe 10 mg tablets (MADG)**

| Constituent | **Examples** | | Function |
|---|---|---|---|
| | **5A** | **5B** | |
| Ezetimibe (17 µm) | 10.00 mg | 10.00 mg | Active |
| Cellulose microcrystalline (Avicel PH200 LM^{™}) | 10.50 mg | 24.00 mg | Filler, binder |
| Lactose spray dried | 55.25 mg | 45.00 mg | Filler |
| Maize starch | 9.75 mg | 7.50 mg | Filler, disintegrant |
| Crospovidone (Polyplasdon XL^{™}) | 1.50 mg | / | Disintegrant |
| Croscarmellose sodium | 6.00 mg | 6.00 mg | Disintegrant |
| Tween 80 (polysorbate) | 1.50 mg | 1.50 mg | Surfactant |
| Macrogol 4000 (PEG-4000) | / | 1.50 mg | Binder |
| Purified water | 4.00 mg | 3.00 mg | Granulation fluid |
| Aerosil 200^{™} (silica, colloidal anhydrous) | 0.50 mg | 0.75 mg | Glidant |
| Magnesium stearat | 1.00 mg | 0.75 mg | Lubricant |

**Table 6. Composition of Ezetimibe 10 mg tablets (A, C, D - MADG; B - wet granulation)**

| Constituent | **Examples** | | | | Function |
|---|---|---|---|---|---|
| | **6A** | **6B** | **6C** | **6D** | |
| Ezetimibe (10 µm) | 10.00 mg | 10.00 mg | 10.00 mg | 10.00 mg | Active |
| Cellulose | 25.25 mg | 27.75 mg | 27.00 mg | 30.00 mg | Filler, binder |
| microcrystalline | | | | | |
| (Avicel PH200 LM^{™}) | | | | | |
| Lactose 70-100 mesh | 42.00 mg | 42.00 mg | 42.00 mg | 42.00 mg | Filler |
| Maize starch | 10.00 mg | 10.00 mg | 10.00 mg | 10.00 mg | Filler |
| Croscarmellose sodium | 4.00 mg | 4.00 mg | 4.00 mg | 4.00 mg | Disintegrant |
| Klucel EXF^{™} | 3.00 mg | 3.00 mg | 3.00 mg | / | Binder, |
| (Hydroxypropylcellulose) | | | | | disintegrant |
| Tween 80 (polysorbate) | 1.75 mg | 1.75 mg | / | / | Surfactant |
| Purified water | 2.50 mg | q.s. | 2.50 mg | 2.50 mg | Granulation fluid |
| Aerosil 200^{™} (silica, | 0.75 mg | 0.75 mg | 0.75 mg | 0.75 mg | Glidant |
| colloidal anhydrous) | | | | | |
| Magnesium stearat | 0.75 mg | 0.75 mg | 0.75 mg | 0.75 mg | Lubricant |

In MADG procedure, the binder was blended with the drug plus filler, a small amount of water (up to 4%) and Tween (up to 5%) were added, and then the mixture was blended thoroughly. Microcrystalline cellulose (MCC), where used, and/or similar ingredient capable of absorbing moisture, was subsequently added to sorb the small amount of moisture present. No traditional drying step was involved. Additionally disintegrants were added and blended. These samples were sieved twice (0.7 mm) and were then mixed with laboratory blender for one min with mixture of magnesium stearate (0.3 mm) and aerosil.

The combination of cellulose microcrystalline (not PH 200 LM), lactose, starch, tween and isomalt with other excipients in small amount during MADG process significantly improved dissolution profile, compared to mixtures for directly compression, dry granulation and wet granulation. On the other hand, mixture of different glidant and lubricant such as colloidal silicon dioxide, and magnesium stearate, did not show positive effect on dissolution of ezetimibe. Also the lactoses with different characteristics (particle size, flowability, compressibility, solubility, porosity) showed radical change in flowabillity and compressibility of powder during tabletting process as well as changes in affinity of ezetimibe to cover and bind mixture on surface of lactose. The spherical nature and particle size assist in the compressibility of the tablet mix. This results in a mix with greatly reduced inter-particle friction, leading to efficient blending and exceptional flow. Good flowability reduces the variation in the tablet weight and hardness. The best results from technological and dissolution point of view was with lactose monohydrate 70-100 mesh and spray dried lactose.

Lactose and/or isomalt combined with starch have a remarkable influence on compressibility (10-20 %) of tablets. Further, dissolution profiles are significantly improved with these two excipients. As further observed, combination of glidants and lubricants with isomalt (GalenlQ721^{™}) or lactose during tabletting process significantly improves flowability (≥ 1.5 g/s; angle of repose 30-40°) of powder with starch and cohesive powder such as ezetimibe.

According to Example 6 (Table 6), influence of different technologies (MADG and wet granulation), as well as influence of Tween 80 and Klucel EXF on dissolution profiles were further investigated. Tween and wet granulation have significant influence on dissolution profile of ezetimibe (see Figure 7). Detailed conditions of tests illustrated in Figure 7 are: 6A - MADG (2.5 % of water) with Tween; 5B - same formulation as 5A but wet granulation; 5C - MADG without Tween; 5D - MADG without Tween and Klucel EXF.

### Examples 7A-7D:

Based on the results of previous Examples, further variations were investigated with a view for improving dissolution profile and stimulators of ezetimibe solubility. Wet granulation and fluid bed granulation were used in these additional investigations alone or in combination with MADG or solid solution formation. Ingredients and technologies are summarized in Table 7.

**Table 7. Composition of Ezetimibe 10 mg tablets (A, B - fluid bed granulation; C - MADG; D - MADG* + fluid bed granulation**)**

| Constituent | **Examples** | | | | Function |
|---|---|---|---|---|---|
| | **7A** | **7B** | **7C** | **7D** | |
| Ezetimibe (10 µm) | 10.00 mg | 10.00 mg | 10.00 mg | 10.00 mg | Active |
| Cellulose microcrystalline | 27.75 | 28.00 | 23.50 | 15.00 mg | Filler, binder |
| (Avicel PH200 LM^{™}) | mg | mg | mg | | |
| Lactose 70-100 mesh | 42.00 mg | / | 42.00 mg | / | Filler |
| Isomalt (galenlQ721^{™}) | / | 45.00 mg | / | 42.00 mg | Filler |
| Maize starch | 10.00 mg | 10.00 mg | 10.00 mg | 10.00 mg | Filler |
| Pluronic F-68^{™} (Poloxamer) | / | / | / | 5.00 mg | Disintegrant |
| Croscarmellose sodium | 4.00 mg | 4.00 mg | 4.00 mg | 5.00 mg | Disintegrant |
| Klucel EXF^{™} (Hydroxypropylcellulose) | 3.00 mg | / | / | / | Binder, disintegrant |
| Tween 80 (polysorbate) | 1.75 mg | 1.50 mg | 3.00 mg | 1.50 mg | Surfactant |
| Purified water | 36.67 mg | 40.00 mg | 6.00 mg | 4* + 40** mg | Granulation fluid |
| Cellulose microcrystalline (Avicel PH 102^{™}) | / | / | / | 10.00 mg | Filler, binder |
| Aerosil 200^{™} (silica, colloidal anhydrous) | 0.75 mg | 0.75 mg | 0.75 mg | 0.75 mg | Glidant |
| Magnesium stearat | 0.75 mg | 0.75 mg | 0.75 mg | 0.75 mg | Lubricant |

Ezetimibe, MCC, lactose or isomalt, starch, croscarmellose sodium, poloxamer and klucel were mixed in high share mixer or fluid bed granulator and water with tween were added throughout nozzle (MADG) or spray nozzle (fluid bed). In MADG avicel, aerosil and magnesium stearate were added after granulation part, sieved (0.7 mm) and then mixture was pressed into tablet form. In fluid bed granulation after ending of spraying water / tween, mass was dried below 2 % of moisture and sieved twice (1 mm and 0.7 mm). Magnesium stearate was added and blended at the end. The resulting final blend was compressed into tablets using a rotary tablet press in a controlled environment.

Influence of variations of technologies and special ingredients and amounts thereof such as Tween, poloxamer, and avicel PH 200 LM, on dissolution profile are shown in Fig. 8.

According to results, technologies like fluid bed granulation and dry granulation in the presence of limited amount of liquid excipient such as surfactant and/or water lead to acceptable dissolution profiles. Avicel PH 200 LM was found to be better used for MADG procedure or *"hard pasta"* granulation (40-50% of water during wet granulation step).

### Examples 8A-8C:

Use of different process technologies and variations in excipients were further investigated as summarized in Table 8.

**Table 8. Composition of Ezetimibe 10 mg tablets (A - MADG + fluid bed granulation; B - wet granulation (pasta); C - wet granulation)**

| Constituent | **Examples** | | | Function |
|---|---|---|---|---|
| **Internals** | **8A** | **8B** | **8C** | |
| Ezetimibe (10 µm) | 10.00 mg | 10.00 mg | 10.00 mg | Active |
| Ethanol (96%) | 40.00 mg | / | 35.00 mg | Granulation fluid |
| Calcium silicate | / | / | 10.00 mg | Disintegrant, porosity agent |
| Texapon K 12^{™} | / | / | 10.00 mg | Surfactant |
| galenlQ721^{™} (isomalt) | 40.00 mg | 40.00 mg | 40.50 mg | Filler |
| Maize starch | 10.00 mg | 10.00 mg | 5.00 mg | Filler |
| Avicel PH 200 LM^{™} (MCC) | 15.00 mg | / | / | Filler |
| Prosolv^{™} (Silicified MCC) | / | / | 15.00 mg | Filler |
| Crospovidone (Polyplasdon XL^{™}) | 1.50 mg | 1.50 mg | 2.00 mg | Disintegrant |
| Croscarmellose sodium | 5.00 mg | 5.00 mg | 4.00 mg | Disintegrant |
| Tween 80 (polysorbate) | 2.50 mg | 2.50 mg | / | Surfactant |
| Purified water | 5.00 mg | 30.00 mg | / | Granulation fluid |
| Constituent | | | | |
| **Externals** | | | | |
| galenlQ721^{™} (isomalt) | 13.50 mg | 13.50 mg | / | Filler |
| Avicel PH 200 LM^{™} (MCC) | / | 15.00 mg | / | Filler |
| Croscarmellose sodium | 1.00 mg | 1.00 mg | 2.00 mg | Disintegrant |
| Aerosil 200^{™} | 0.75 mg | 0.75 mg | 0.75 mg | Glidant |
| Magnesium stearat | 0.75 mg | 0.75 mg | 0.75 mg | Lubricant |

In examples 8A and 8C ezetimibe was dissolved in ethanol (250 mg/mL in ethanol) and thereafter sprayed through nozzle on the surface of excipients. The ingredients were weighted and blended in laboratory blender purpose for 5 min. Subsequently, mixture of powders (internals) was sprayed with solution made of ezetimibe / ethanol or ezetimibe / SDS / Ca-silicate / ethanol. Afterwards the mixtures were dried; sieved (0.7 mm, twice) and external excipients were added. The resulting final blend was compressed into tablets using a rotary tablet press in a controlled environment.

Use or calcium silicate, sodium dodecyl sulfate and ethanol provided for enhanced prevention of agglomeration of ezetimibe during granulation process, and made the final tablet to have more porosity.

During the procedure described in example 8B, internal excipients and ezetimibe were first of all blended in high share mixture, and then tween was added as well as purified water. After adding of approximately 40-45% of water calculating on dry mass, we got pasta of ezetimibe. Avicel PH 200 LM and isomalt have been added to take excessively amount of water and mixture was ready for drying step in fluid bed till 3% of moisture. Afterward, mixtures were dried; sieved (0.7 mm, twice) and lubricant and gliant were added.

With a view to positive effects on dissolution profiles, further investigations were made.

### Examples 9A-9C:

Solid solutions were investigated in the next stage.

**Table 9. Composition of Ezetimibe solid solution**

| Constituent | **Examples** | | | Function |
|---|---|---|---|---|
| | **9A** | **9B** | **9C** | |
| Ezetimibe (10 µm) | 10.00 mg | 10.00 mg | 10.00 mg | Active |
| Macrogol 4000 (PEG-4000) | 20.00 mg | / | / | Solid solution agent |
| PVP K-25 (povidone) | / | 20.00 mg | / | Solid solution agent |
| PVA (Polyvinyl alcohol) | / | / | 20.00 mg | Solid solution agent |
| Ethanol (96%) | 40.00 mg | 40.00 mg | 40.00 mg | Solvent |

Ezetimibe and solid solution agent were simply dissolved in ethanol and then solvent was evaporated at elevated temperature (45-50°C). After evaporation of ethanol we got solid solution of ezetimibe in PEG and PVP. PVA made strong binding character between particles.

With suitable solid solution agents, illustrated here with a selection of the excipients polyethylene glycol 4000 (Fig. 9a) and polyvinylpyrrolidon (PVP K-25; Fig. 9b), physical status of ezetimibe in solid solution was confirmed. Therefore, solid solution provides a useful alternative to wet granulation and other technologies. Moreover, further tests have shown that suitable binders can enhance binding properties between ezetimibe particles; for example, polyvinyl alcohol (PVA) has been found to act as a suitable binder to be preferably combined with ezetimibe.

Formation of solid solution with appropriate solid solid agent, here PEG and PVP, have shown positive influence on increasing solubility of ezetimibe.

### Examples 10A and 10B:

Formation of solid solution was used in further investigation and compared with wet granulation. Experimental setup is summarized in Table 10 below.

**Table 10. Composition of Ezetimibe 10 mg tablets (A - wet granulation; B - solid solution + fluid bed granulation)**

| Constituent | **Examples** | | Function |
|---|---|---|---|
| **Internals** | **10A** | **10B** | |
| Ezetimibe (10 µm) | 10.00 mg | 10.00 mg | Active |
| Ethanol (96%) | / | 60.00 mg | Granulation fluid/Solvent |
| Texapon K 12^{™} | 5.00 mg | 4.00 mg | Surfactant |
| Lactose 70-100 mesh | 64.75 mg | 64.75 mg | Filler |
| Maize starch | 5.00 mg | 5.00 mg | Filler |
| Crospovidone (Polyplasdon XL^{™}) | / | 1.00 mg | Disintegrant |
| Croscarmellose sodium | 8.00 mg | 6.00 mg | Disintegrant |
| PVP K-25 (povidone) | 6.00 mg | 6.00 mg | Binder/ Solid solution agent |
| Purified water | 30.00 mg | 10.00 mg | Granulation fluid |
| Constituent | | | |
| **Externals** | | | |
| Croscarmellose sodium | / | 2.00 mg | Disintegrant |
| Aerosil 200^{™} | 0.50 mg | 0.50 mg | Glidant |
| Magnesium stearat | 0.75 mg | 0.75 mg | Lubricant |

Wet granulation was performed on the same way as it was described for the other samples with the same technology. Solid solution fluidization is interesting according to this part of investigation. Texapone was dissolved in water and ezetimibe / PVP in ethanol. After blending of starch, lactose, croscarmellose sodium, and crospovidone solution of texapone was sprayed on excipients. Next step was drying in fluid bad and after active solution was sprayed on excipients in fluid bed granulator. Attention was paid that moisture of granulates was below 1.5 %. Mass was then sieved twice through 0.7 mm sieve. Final mixture was blended with external ingredients and prepared for tableting.

Dissolution profile was similar to results for solid solution (Example 9), but could be improved by Nonaqueos Moisture Activated Dry Granulation (NAMAGD) with Tween 80 as a surfactant and granulation agent, as will be described in Example 11 below.

### Example 11:

Investigations were carried out with Tween 80 content of 1.0 to 10.0 % (w/w). The selection of these Tween contents was based on former experiments for MADG model in which water contents of 2.0 to 4.0 % were used. The best quality of tablets as well as disintegration properties (≤ 5 min) was achieved with 2.5 % of Tween 80.

**Table 11. Composition of Ezetimibe 10 mg tablets (NAMADG)**

| Constituent | **Example 11** | Function |
|---|---|---|
| Ezetimibe (10 µm) | 10.00 mg | Active |
| Lactose spray dried | 70.50 mg | Filler |
| Maize starch | 5.00 mg | Filler, disintegrant |
| Crospovidone (Polyplasdon XL^{™}) | 1.00 mg | Disintegrant |
| Croscarmellose sodium | 6.00 mg | Disintegrant |
| Tween 80 (polysorbate) | 2.50 mg | Surfactant |
| Klucel EXF^{™} (Hydroxypropylcellulose) | 3.50 mg | Binder, Disintegrant |
| Aerosil 200^{™} (silica, colloidal anhydrous) | 0.75 mg | Glidant |
| Magnesium stearat | 0.75 mg | Lubricant |

Lactose SD and starch were blended firstly alone and then with ezetimibe. Mixture was sieved throughout sieve 0.7 mm, and disintegrants were added and mixed. Tween was sprayed on excipients with active principle. Granulation was done with high share mixing and chopping. Granulate was sieved twice (0.7 mm). After added of klucel, aerosil and magnesium stearate, mass was prepared for tableting. Dissolution profile of this very simple, economical, and robust sample was good (Figure 10). Positive influence of lactose, starch, and tween was confirmed to reach very good dissolution profile of ezetimibe.

### Example 12:

Based on experiences with MADG and NAMADG, alternative investigations were carried out with a combination of limited amount of water and limited amount of non-aqueous liquid excipient (tested with Tween 80 as surfactant), named DMADG.

**Table 12. Composition of Ezetimibe 10 mg tablets (DMADG).**

| Constituent | **Example 12** | Function |
|---|---|---|
| Ezetimibe (10 µm) | 10.00 mg | Active |
| Lactose spray dried | 69.00 mg | Filler |
| Maize starch | 5.00 mg | Filler, disintegrant |
| Crospovidone (Polyplasdon XL) | 1.50 mg | Disintegrant |
| Croscarmellose sodium | 6.00 mg | Disintegrant |
| Tween 80 (polysorbate) | 2.50 mg | Surfactant and moisture activator |
| Purified water | 2.00 mg | Moisture activator |
| Klucel EXF^{™} (Hydroxypropylcellulose) | 3.00 mg | Binder, Disintegrant |
| Aerosil 200^{™} (silica, colloidal anhydrous) | 0.60 mg | Glidant |
| Magnesium stearat | 0.40 mg | Lubricant |

Lactose SD and starch were blended firstly alone and then with ezetimibe, and after the 3 minutes disintegrants were added and mixed for another 1 minute. Firstly, water was sprayed on excipients with active principle to potent binders between hydrophilic compound and API. The second activation was done by spraying of Tween on water activated mixture of excipients and active principle. Granulation was done with high share mixing and chopping (200-250 rpm for 3 minutes). Granulate was sieved (0.7 mm). After added of klucel, aerosil and magnesium stearate, mass was prepared for tableting. Dissolution profile of this very simple, economical, and robust sample was acceptable and even better in first point (5 minute) then results shown in Figure 10.

### Example 13:

In this Example, positive influence of hydrophilic excipients after blending with API was tested and confirmed also in the final compressed tablets. Spray dried lactose or isomalt was used as particulate saccharide excipients. Further, limited amount of water for moisture activation of dry granulation, with or without further activation by limited amount of non-aqueous liquid excipient (Tween 80 as surfactant) was tested.

Granulation procedure is the similar to those used for examples 11 and 12.

**Table 13. Composition of Ezetimibe 10 mg tablets (DMADG and influence of different hydrophilic excipients).**

| Constituent | **Examples** | | | | Function |
|---|---|---|---|---|---|
| | **13A** | **13B** | **13C** | **13D** | |
| Ezetimibe (10 µm) | 10.00 mg | 10.00 mg | 10.00 mg | 10.00 mg | Active |
| Lactose SD* | 70.35 mg | 75.35 mg | 81.00 mg | 81.00 mg | Filler |
| Maize starch | 5.00 mg | / | / | / | Filler |
| Croscarmellose sodium | 6.00 mg | 6.00 mg | 6.00 mg | / | Disintegrant |
| Primojel^{™} (Sodium starch glycolate) | / | / | / | 6.00 mg | Disintegrant |
| Klucel EXF^{™} (Hydroxypropylcellulose) | 3.00 mg | 3.00 mg | / | / | Binder, disintegrant |
| Tween 80 (polysorbate) | 2.50 mg | 2.50 mg | / | / | Surfactant - activator |
| Purified water | 2.00 mg | 2.00 mg | 2.00 mg | 2.00 mg | Activator |
| Aerosil 200^{™} (silica, colloidal anhydrous) | 0.75 mg | 0.75 mg | 0.60 mg | 0.60 mg | Glidant |
| Magnesium stearat | 0.40 mg | 0.40 mg | 0.40 mg | 0.40 mg | Lubricant |

| | | | | | |
|---|---|---|---|---|---|
| *Examples 13E-13H are the same as 13A-13D but isomalt was used instead of lactose | | | | | |

All Examples 13A to 13H provided very simple, economical, and robust samples. Good results on dissolution profiles were achieved as shown in Figure 11.

## Claims

1. A formulation of ezetimibe, comprising ezetimibe in the form of primary particles and a hydrophilic excipient in particulate form having said ezetimibe particles adsorbed to.

2. The formulation of ezetimibe according to claim 1, wherein the hydrophilic excipient is saccharide, preferably mono- or disaccharide.

3. A formulation of ezetimibe, comprising ezetimibe combined with at least two types of hydrophilic excipients, wherein one type of hydrophilic excipient is a starch and the other type of hydrophilic excipient is a saccharide selected from mono-saccharides, disaccharides and cellulose substances.

4. The formulation of ezetimibe according to claim 2 or 3, wherein the saccharide is selected from lactose and isomalt.

5. A formulation of ezetimibe comprising ezetimibe, granulated in admixture with up to 8 wt.-%, preferably up to 6 wt.-% and more preferably up to 4 wt.-% of water or an aqueous liquid, and/or with up to 10 wt.%, preferably 1 to below 10 wt.% of nonaqueous excipient selected from liquid substances.

6. The formulation according to claim 5, wherein the nonaqueous excipient is selected from liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol.

7. A formulation of ezetimibe comprising ezetimibe, admixed in solid solution with a solid solution agent.

8. The formulation according to claim 7, wherein the solid solution agent is selected from polyethylene glycol and polyvinylpyrrolidone.

9. The formulation according to any one of the preceding claims, further comprising a solid excipient selected from microcrystalline cellulose, lactose, isomalt, mannitol, sorbitol, starch, calcium phosphates, calcium or sodium carboxymethylcellulose, cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate, preferably selected from solid polyethylene glycol, isomalt, microcristalline cellulose, mannitol and lactose, preferably comprising at least starch, lactose, isomalt, PEG, PVP and Tween, optinally also croscarmellose sodium.

10. The formulation of ezetimibe according to any one of the preceding claims, wherein the ezetimibe particles have a particle size d(0.9) of 40µm or lower, preferably d(0.9) of 25µm or lower, and/or wherein said particulate hydrophilic excipient has a mean particle size relatively larger than that of ezetimibe particles, preferably wherein said particulate hydrophilic excipient has a mean particle size from 10 to 400 µm.

11. A process for producing a formulation of ezetimibe using dry granulation in the absence of granulation liquid or in the presence of limited amount of water or aqueous liquid of up to 8 wt.-%, and/or in the presence of limited amount of non-aqueous liquid of up to 10 wt.-%, or using solid solution formation of ezetimibe.

12. The process according to claim 11, wherein dry granulation is carried out in the presence of limited amount of non-aqueous liquid which is selected from liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol.

13. A pharmaceutical formulation, comprising a formulation of ezetimibe according to any one of claims 1 to 10, or comprising a formulation of ezetimibe obtained by a process according to claim 11 or 12.

14. The pharmaceutical formulation according to claim 13, which has a dissolution profile by releasing ezetimibe at a rate of higher than 30 %, preferably higher than 40 %, and more preferably at least 60 %, preferably at least 60 %, within 30 minutes, relative to the original amount of eprosartan in the formulation, tested using USP apparatus 2, placing the formulations in 1000 ml 0.1 M hydrochloric acid at 37 ± 0.5°C with paddle speed of 50 rpm.

15. The formulation according to any of claims 1 to 10, or pharmaceutical formulation according to claim 13 or 14 for use in the prophylaxis or treatment of cholesterol-associated diseases.
